(19) Europäisches Patentamt
European Patent Office
Office européen des brevets

(11) Veröffentlichungsnummer: **0 071 135**
**A1**

(12) **EUROPÄISCHE PATENTANMELDUNG**

(21) Anmeldenummer: **82106471.4**

(22) Anmeldetag: **19.07.82**

(51) Int. Cl.³: **C 07 C 47/55**
C 07 C 45/41

(30) Priorität: **28.07.81 DE 3129651**

(43) Veröffentlichungstag der Anmeldung:
**09.02.83 Patentblatt 83/6**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Gallenkamp, Bernd, Dr.**
**Claudiusweg 5**
**D-5600 Wuppertal 1(DE)**

(54) **Verfahren zur Herstellung von 3-Brom-4-fluor-benzaldehyd.**

(57) Die vorliegende Erfindung betrifft ein Verfahren zur Herstellung von 3-Brom-4-fluor-benzaldehyd indem man 3-Brom-4-fluor-benzoesäureester mit Natrium-bis-(2-methoxy-ethoxy)-aluminiumhydrid reduziert.

EP 0 071 135 A1

BAYER AKTIENGESELLSCHAFT 5090 Leverkusen, Bayerwerk

0071135

Zentralbereich
Patente, Marken und Lizenzen Rt/AB

# Verfahren zur Herstellung von 3-Brom-4-fluor-benzaldehyd

Die vorliegende Erfindung betrifft ein neues Verfahren zur Herstellung von 3-Brom-4-fluor-benzaldehyd.

Es ist bereits bekannt, daß es möglich ist, Aldehyde durch Umsetzung von Carbonsäureestern mit Natrium-bis-(2-methoxy-ethoxy)-aluminiumhydrid herzustellen (vergleiche Synthesis 1976, 525). Weiterhin ist bekannt, daß halogensubstituierte Verbindungen durch Umsetzung mit Natrium-bis-(2-methoxy-ethoxy)-aluminiumhydrid enthalogeniert werden (vergleiche THL 1968, 33o4).

Es wurde gefunden, daß man 3-Brom-4-fluor-benzaldehyd der Formel

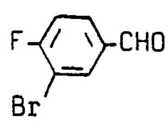

(I)

erhält, wenn man 3-Brom-4-fluor-benzoesäureester der Formel

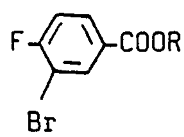

(II)

in welcher

R für $C_1$-$C_4$-Alkyl steht,

mit Natrium-bis-(2-methoxy-ethoxy)-aluminiumhydrid, in Gegenwart von heterocyclischen Aminen, in einer Schutzgasatmosphäre und unter Verwendung von Verdünnungsmitteln bei Temperaturen zwischen -loo und 4o°C umsetzt.

Es ist als ausgesprochen überraschend zu bezeichnen, daß nach dem erfindungsgemäßen Verfahren 3-Brom-4-fluor-benzaldehyd in hohen Ausbeuten entsteht, da nach dem Stand der Technik eine Entbromierung der Verbindung mit der Formel II unter Bildung von 4-Fluor-benzoesäureester und/oder 4-Fluorbenzaldehyd zu erwarten war.

Die erfindungsgemäße Reaktion kann durch folgendes Formelschema skizziert werden:

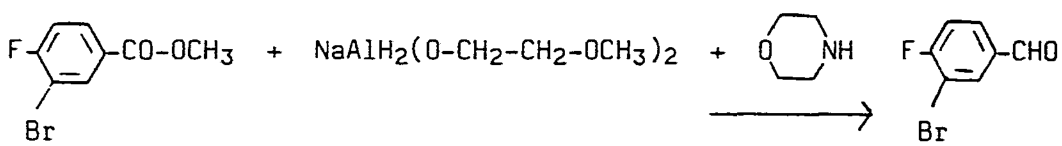

Die als Ausgangsverbindungen einzusetzenden 3-Brom-4-fluor-benzoesäureester der Formel (II) sind Gegenstand einer eigenen älteren nicht vorveröffentlichten Patentanmeldung. Die Herstellung von 3-Brom-4-fluor-benzoesäureestern der Formel (II) erfolgt danach durch Umsetzung von 3-Brom-4-fluorbenzoesäure oder deren reaktionsfähige Derivate mit Alkoholen gegebenenfalls in Gegenwart von Katalysatoren wie z.B. Schwefelsäure bei Temperaturen zwischen 2o und loo°C (vergleiche Deutsche Patentanmeldung P 3 o36 967 [Le A 2o 579]).

Als Beispiele für die Verbindungen der Formel II seien im einzelnen genannt:
3-Brom-4-fluor-benzoesäure-methyl-, -ethyl-, -n-propyl-, -iso-propyl-, -n-butyl-, -iso-butyl-, -sec.-butyl- und tert.-butyl-ester.

Das erfindungsgemäße Verfahren zur Herstellung von 3-Brom-4-fluor-benzaldehyd (I) wird unter Verwendung von inerten organischen Lösungsmitteln wie z.B. Toluol, Xylol, Ether wie Diethyl- und Dibutylether, Glycoldimethylether und Diglycoldimethylether, Tetrahydrofuran und Dioxan durchgeführt.

Als inertes Schutzgas wird Argon oder Stickstoff verwendet.

Das erfindungsgemäße Verfahren wird in Gegenwart von heterocyclischen Aminen wie z.B. Morpholin oder N-Methylpiperazin durchgeführt.

Die Reaktionstemperatur liegt beim erfindungsgemäßen Verfahren zwischen -loo und +4o°C, vorzugsweise zwischen -6o und +3o°C.

Die Umsetzung wird im allgemeinen bei Normaldruck durchgeführt.

Auf 1 Mol 3-Brom-4-fluorbenzoesäureester der Formel (II) setzt man zwischen 3 bis 7 Mol, vorzugsweise zwischen 5 und 6 Mol Natrium-bis-(2-methoxy-ethoxy)-aluminiumhydrid und 4 bis 8 Mol, vorzugsweise 6 bis 7 Mol N-Methylpiperazin bzw. Morpholin ein.

Eine Mischung aus Reduktionsmittel und Verdünnungsmittel wird unter Kühlung und unter Schutzgas mit einer Lösung aus Amin und Verdünnungsmittel versehen. Diese Reduktionsmischung wird unter Rühren bei der erforderlichen Temperatur vorsichtig zu einer Lösung aus 3-Brom-4-fluor-benzoesäureester der Formel (II) und Verdünnungsmittel gegeben. Diese Reaktion wird ebenfalls in einer Schutzgasatmosphäre durchgeführt. Das Reaktionsgemisch wird bis zum Ende der Umsetzung

gerührt, wobei die Temperatur auf 20 bis 25°C ansteigt.

Zur Aufarbeitung versetzt man mit Wasser und verdünnter Schwefelsäure, trennt die organische Phase ab, extrahiert die wäßrige Phase noch mit Toluol, die vereinigten organischen Phasen werden z.B. mit Bicarbonatlösung gewaschen, getrocknet und das Lösungsmittel abdestilliert.

Als Reinheitskriterium dient das Gaschromatogramm.

Der nach dem erfindungsgemäßen Verfahren herzustellende 3-Brom-4-fluor-benzaldehyd (I) kann als Zwischenprodukt zur Herstellung von insektizid und akarizid wirksamen Pyrethroiden verwendet werden (vergleiche DE-OS 2 933 279). So erhält man daraus beispielsweise die entsprechenden Acetale durch Umsetzung mit Alkan(di)olen wie z.B. 1,2-Ethandiol in Gegenwart eines Wasserbindemittels wie z.B. Chlortrimethylsilan, gegebenenfalls in Gegenwart von Verdünnungsmitteln wie z.B. Toluol und Katalysatoren wie z.B. Chlorwasserstoffsäure bei Temperaturen zwischen 2o und 12o°C; die auf diese Weise erhaltenen 3-Brom-4-fluor-benzaldehydacetale werden mit Phenolaten wie z.B. Natriumphenolat in Gegenwart von Katalysatoren wie z.B. Kupfer-I-oxid und in Gegenwart von Verdünnungsmitteln wie z.B. Diglyme (Bis-(2-methoxyethyl)-ether ) sowie gegebenenfalls in Gegenwart von Hilfsstoffen wie z.B. Kaliumchlorid und gegebenenfalls in Gegenwart eines basischen Entwässerungsmittels wie z.B. Natriumhydrid bei Temperaturen zwischen 13o und 17o°C umgesetzt. Die anschließende Acetalspaltung der 4-Fluor-3-phenoxybenzaldehyd-acetale kann nach üblichen Methoden durchgeführt werden; man erhält auf diese Weise 4-Fluor-3-phenoxy-benzaldehyd, welcher als Zwischenprodukt für Pyrethroide bekannt ist (vergleiche DE-OS 2 7o9 264).

Beispiel 1

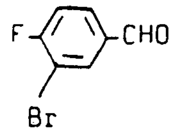

In 161,7 g (o,56 Mol) 7o%ige Natrium-bis-(2-methoxy-ethoxy)-aluminiumhydridlösung in 2oo ml Toluol tropft man bei 0°C 54,8 g (o,63 Mol) Morpholin in 2oo ml Toluol unter Argon zu. Diese Reduktionsmischung wird unter Rühren bei -5o°C zu einer Lösung von 23,3 g (o,1 Mol) 4-Fluor-3-brom-benzoesäuremethylester in 2oo ml Toluol getropft. Das Reaktionsgemisch wird 16 Stunden gerührt; die Temperatur steigt dabei auf etwa 2o bis 25°C an. Nach Versetzen mit 3oo ml Wasser und verdünnter Schwefelsäure wird die organische Phase abgetrennt. Die wässrige Phase wird noch zweimal mit jeweils loo ml Toluol extrahiert, die vereinigten organischen Phasen werden mit Natriumhydrogencarbonatlösung und Wasser gewaschen, getrocknet und anschließend wird Toluol abdestilliert.

Man erhält auf diese Weise 18,3 g eines Produktes. Nach der Gaschromatographischen Analyse besteht dieses aus

76,3 % 3-Brom-4-fluor-benzaldehyd (69% der Theorie)
12,6 % 4-Fluorbenzaldehyd
4,3 % Toluol
3,6 % 4-Fluor-benzoesäuremethylester
3,2 % 4-Fluor-3-brombenzylalkohol

Patentanspruch

1. Verfahren zur Herstellung von 3-Brom-4-fluor-benzaldehyd der Formel

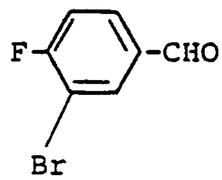

(I)

indem man 3-Brom-4-fluor-benzoesäureester der Formel

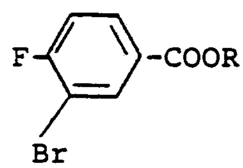

(II)

in welcher
R für $C_1$-$C_4$-Alkyl steht,

mit Natrium-bis-(2-methoxy-ethoxy)-aluminiumhydrid, in Gegenwart von heterocyclischen Aminen, in einer Schutzgasatmosphäre und unter Verwendung von Verdünnungsmitteln bei Temperaturen zwischen -100 und 40°C umsetzt.

Europäisches Patentamt

# EUROPÄISCHER RECHERCHENBERICHT

0071135
Nummer der Anmeldung

EP 82 10 6471.4

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| Y | DE - A1 - 2 933 979 (BAYER)<br>* Anspruch 7 *<br>-- | 1 | C 07 C 47/55<br>C 07 C 45/41 |
| Y | US - A - 3 660 416 (J. VIT)<br>* Beispiele 1, 6 *<br>-- | 1 | |
| Y | US - A - 3 839 367 (J. VIT)<br>* Beispiele 1, 6 *<br>---- | 1 | |
| | | | RECHERCHIERTE SACHGEBIETE (Int. Cl.³) |
| | | | C 07 C 45/41<br>C 07 C 47/55 |
| | | | KATEGORIE DER GENANNTEN DOKUMENTE |
| | | | X: von besonderer Bedeutung allein betrachtet<br>Y: von besonderer Bedeutung in Verbindung mit einer anderen Veroffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsatze<br>E: alteres Patentdokument, das jedoch erst am oder nach dem Anmeldedatum veroffentlicht worden ist<br>D: in der Anmeldung angefuhrtes Dokument<br>L: aus andern Grunden angefuhrtes Dokument |
| X | Der vorliegende Recherchenbericht wurde fur alle Patentanspruche erstellt | | &: Mitglied der gleichen Patentfamilie, ubereinstimmendes Dokument |

| Recherchenort | Abschlußdatum der Recherche | Prufer |
|---|---|---|
| Berlin | 01-11-1982 | KNAACK |

EPA form 1503.1 06.78